**Europäisches Patentamt**

**European Patent Office**

**Office européen des brevets**

(19)

(11) Veröffentlichungsnummer: **0 083 924**
**B1**

(12) **EUROPÄISCHE PATENTSCHRIFT**

(45) Veröffentlichungstag der Patentschrift:
04.11.87

(51) Int. Cl.⁴: **C 07 C 87/60**, C 07 C 85/04

(21) Anmeldenummer: **83100032.8**

(22) Anmeldetag: **04.01.83**

(54) Verfahren zur Herstellung von 2-Amino-5-nitrotoluol.

(30) Priorität: **08.01.82 DE 3200308**

(43) Veröffentlichungstag der Anmeldung:
**20.07.83 Patentblatt 83/29**

(45) Bekanntmachung des Hinweises auf die Patenterteilung:
**04.11.87 Patentblatt 87/45**

(84) Benannte Vertragsstaaten:
**BE CH DE FR GB IT LI**

(56) Entgegenhaltungen:
**EP-A-0 004 623**
**DE-A-2 720 316**
**US-A-2 459 002**
**US-A-3 277 175**
**US-A-3 313 854**

**Chemical Abstracts Band 94, Nr. 9, 2. März 1981,**
**Columbus, Ohio, USA; S.A. KONDRATOV et al.**
**"Catalysis by copper complexes in the reaction of**
**o-nitrochlorobenzene with ammonia", Seite 646,**
**Spalte 2, Abstract Nr. 64770z**
**Houben-Weyl "Methoden der organischen**
**Chemie", Bd. XI/I (1957), Seiten 63,64**

(73) Patentinhaber: **HOECHST AKTIENGESELLSCHAFT,**
**Postfach 80 03 20, D-6230 Frankfurt am Main 80**
**(DE)**

(72) Erfinder: **Papenfuhs, Theodor, Dr., Heinrich-**
**Bleicher- Strasse 40, D-6000 Frankfurt am Main 50**
**(DE)**
Erfinder: **Berthold, Rüdiger, Dr., Geierfeld 55,**
**D-6232 Bad Soden am Taunus (DE)**

EP 0 083 924 B1

## Beschreibung

2-Amino-5-nitrotoluol dient als wertvolle Diazokomponente zur Erzeugung von Azoverbindungen, beispielsweise von wasserunlöslichen Azofarbstoffen auf der Faser nach der Methode der Eisfarbentechnik.

Als einzige technisch durchführbare Synthese ist bisher aus BIOS Final Report 986/2; No. 167, S. 297 - 306 bekannt, 2-Aminotoluol mit Benzolsulfochlorid zu Benzolsulfonsäure-(2-methyl-phenyl)-amid zu acylieren, dieses in Chlorbenzol zu nitrieren und abschließend unter hydrolytischer Abspaltung der Schutzgruppe das 2-Amino-5-nitrotoluol zu isolieren. Hierbei liegt die Ausbeute bei ca. 75 % d. Th.. Abgesehen von dieser unbefriedigenden Ausbeute, den zahlreichen Verfahrensstufen und dem damit verbundenen Chemikalienaufwand birgt dieses Verfahren weitere schwerwiegende Nachteile, die seine technische Realisierung aus verfahrenstechnischer und ökologischer Sicht problematisch machen:

Die Nitrierung im Lösemittel erfordert einen hohen sicherheitstechnischen Aufwand, da zündbare Gasgemische entstehen können. Außerdem sind zur erforderlichen Lösemittel-Regeneration gesonderte spezielle Apparate notwendig.

Die hydrolytische Abspaltung der Schutzgruppe unter Bildung von Benzolsulfonsäure kann nur mit einem großen Überschuß konzentrierter Schwefelsäure erfolgen, aus der das gewünschte 2-Amino-5-nitrotoluol durch Filtration isoliert wird. Neben dem zur Abtrennung aus Schwefelsäure erforderlichen hohen apparativen Aufwand resultiert dabei ein organisch hochbelastetes saures Filtrat, da die gesamte Benzolsulfonsäure in der überschüssig aufgewandten Schwefelsäure gelöst bleibt und nicht regeneriert werden kann. Dessen Entsorgung ist nur durch Verbrennen in einer säurefesten Anlage möglich, da es biologisch auf Grund der hohen organischen und anorganischen Last nicht abbaubar ist.

Es bestand daher ein Bedarf an einem Verfahren zur Herstellung von 2-Amino-5-nitrotoluol, das die aufgezeigten Nachteile weitgehend vermeidet und in hohen Ausbeuten technisch und ökologisch vorteilhaft durchführbar ist.

Es wurde nun gefunden, daß man 2-Amino-5-nitrotoluol in nahezu quantitativer Ausbeute durch Umsetzung von 2-Chlor-5-nitrotoluol mit wäßrigem Ammoniak einer Konzentration von mindestens 30 Gew.-% herstellen kann.

Die Umsetzung verläuft bei Temperaturen ab etwa 180° C mit ausreichender Geschwindigkeit. Temperaturen oberhalb von etwa 250° C sind wegen der entstehenden Drücke weniger vorteilhaft. Bevorzugt ist eine Umsetzungstemperatur von 200 - 220° C. Zur Beschleunigung der Reaktion bzw. zur Senkung der Umsetzungstemperatur können katalytische Mengen Kupfer oder Kupferverbindungen zugegeben werden. Das Ammoniak wird im Überschuß angewandt. Nicht umgesetztes Ammoniak kann in den Prozeß zurückgeführt werden. Es entsteht hierbei als Nebenprodukt lediglich 1 Mol Ammoniumchlorid.

Die Konzentration des eingesetzten Ammoniaks beträgt vorteilhaft bis zu 80, vorzugsweise 40 bis 70, Gew.-%. Die Umsetzung kann diskontinuierlich und kontinuierlich durchgeführt werden. Wegen der auftretenden Drücke von 60-100 bar kann eine kontinuierliche Umsetzung, beispielsweise in von außen beheizten Rohrreaktoren, infolge des geringeren Raum- und damit Apparatebedarfs von Vorteil sein.

Das als Ausgangsprodukt benötigte 2-Chlor-5-nitrotoluol ist durch Nitrierung von o-Chlortoluol zugänglich (Rec. Trav. Chim. Pays-Bas 32, (1913) 244 ff.). Aus dem dabei entstehenden Isomerengemisch ist es durch Destillation in 43 % Ausbeute isolierbar (a.a.o. 286). Als Vorlauf fällt dabei reines 2-Chlor-6-nitrotoluol an; die restlichen Isomeren sind in bekannter Weise zu trennen. Da alle Isomeren auf Grund ihrer reaktiven Gruppen vielseitig einsetzbare Zwischenprodukte darstellen, für die ausreichender Bedarf besteht, fallen bei dieser Reaktion keine unerwünschten Nebenprodukte an.

Auch unter Einbeziehung dieser Vorstufe stellt somit diese Erfindung eine außerordentlich günstige Verfahrensweise dar.

Das erfindungsgemäße Verfahren erlaubt es somit, 2-Amino-5-nitrotoluol wesentlich einfacher als nach dem bisher bekannten Verfahren herzustellen, wobei keine unerwünschten Nebenprodukte anfallen. Die Qualität des Produkts erlaubt seinen Einsatz zur Herstellung von Azoverbindungen ohne weitere Reinigung.

Aus HOUBEN-WEYL, Band XI/1 (1957), Seiten 63/64 ist die Umsetzung von Nitrochlorbenzolen mit wäßrigem Ammoniak einer Konzentration von 27 % und mehr prinzipiell bekannt. aus Chemical Abstracts, Band 94, Nr. 9 (1981), Seite 646, Abstract Nr. 64 770z ist ferner bekannt, daß die Umsetzung von o-Nitrochlorbenzol mit Amoniak durch Kupfer oder Kupferverbindungen katalysiert wird. Aus der erstgenannten Literaturstelle ist aber auch bekannt (vgl. Tabelle 10 auf Seite 64), daß ein zur Nitrogruppe o-ständiges Chloratom erheblich leichter nucleophil substituiert werden kann als ein p-ständiges. So kann das im 2,5-Dichlornitrobenzol 2-ständige Chloratom durch eine Aminogruppe mittels wäßrigem Ammoniak ausgetauscht werden, wobei das 2-Nitro-4-chloranilin in 99 %iger Ausbeute erhalten wird (vgl. Tabellenbeispiel 4). Dagegen ist aus den Tabellenbeispielen 1 und 2 ersichtlich, daß die Umsetzung von p-Chlorbenzol mit wäßrigem Ammoniak unter Austausch des Chloratoms durch eine Aminogruppe nur unter technisch praktisch nicht mehr realisierbaren bzw. sicherheitstechnisch nicht mehr vertretbaren Bedingungen möglich ist. Im Hinblick darauf, daß beim erfindungsgemäßen Verfahren ein p-Nitrochlorbenzol umgesetzt wird, welches am aromatischen Kern eine den Chloraustausch desaktivierende Methylgruppe enthält, war zu erwarten, daß bei dieser Verbindung der Chloraustausch nur unter noch extremeren, technisch nicht realisierbaren Bedingungen erreicht werden kann. Es war daher überraschend, daß die Umsetzung von 2-Chlor-5-nitrotoluol mit höher bis hochprozentigem wäßrigen Ammoniak - sowohl in Gegenwart als auch in Abwesenheit von Kupfer oder Kupferverbindungen selektivem Chloraustausch in hohen Ausbeuten und auf ökologisch vorteilhafte Weise zum 2-Amino-5-nitrotoluol führt.

In den folgenden Beispielen beziehen sich Prozentangaben auf das Gewicht, sofern nichts anderes angegeben ist.

**Beispiel 1**

In einem 1 l-Stahlautoklav erhitzte man ein Gemisch aus 86 g 2-Chlor-5-nitro-toluol, 300 g 25 %iger wäßriger Ammoniaklösung, 1 g basischem Kupfercarbonat und 100 g flüssigem Ammoniak 12 Stunden unter gutem Rühren auf 200° C. Dabei baute sich ein Druck von 90 bar auf. Danach wurde auf Raumtemperatur abgekühlt und überschüssiges Ammoniak abgeblasen. Nun wurde die Reaktionslösung über eine Nutsche gesaugt. Als Rückstand erhielt man 71,5 g 2-Amino-5-nitrotoluol, welches bei 60° C im Vakuumtrockenschrank getrocknet wurde. Die Ausbeute betrug 68,4 g, entsprechend 90 % d. Theorie; Fp 118 - 122° C. Nach gaschromatographischem Untersuchen enthielt das Produkt 98,7 bis 99,8 % 2-Amino-5-nitrotoluol, kein 2-Chlor-5-nitrotoluol (Ausgangsprodukt) und nur 0,2 bis 1,3 % später eluierbare Komponenten.

**Beispiel 2**

172 g 2-Chlor-5-nitrotoluol wurden mit 300 g 25 %iger wäßriger Ammoniaklösung und 150 g flüssigem Ammoniak 12 Stunden auf 200° C erhitzt. Dabei entstand ein Druck von ca. 100 bar. Nach dem Abkühlen auf Raumtemperatur und Entspannen des Autoklaven wurde die Reaktionsmischung abgesaugt. Man erhielt 78,5 g Feuchtprodukt und nach dem Trocknen im Vakuumtrockenschrank bei 60° C 72,0 g trockenes 2-Amino-5-nitrotoluol (Fp 120 - 123° C), entsprechend 94,7 % der Theorie. Die Zusammensetzung dieses Rohproduktes entsprach nach gaschromatographischer Untersuchung der in Beispiel 1 angegebenen.

**Patentansprüche**

1. Verfahren zur Herstellung von 2-Amino-5-nitrotoluol, dadurch gekennzeichnet, daß man 2-Chlor-5-nitrotoluol mit wäßrigem Ammoniak einer Konzentration von mindestens 30 Gew.-% umsetzt.

2. Verfahren nach Anspruch 1, dadurch gekennzeichnet, daß die Umsetzung bei 180 - 250° C erfolgt.

3. Verfahren nach Anspruch 1 und 2, dadurch gekennzeichnet, daß die Umsetzung bei 200 - 220° C erfolgt.

4. Verfahren nach Anspruch 1 bis 3, dadurch gekennzeichnet, daß die Konzentration des eingesetzten Ammoniaks bis zu 80 Gew.-% beträgt.

5. Verfahren nach Anspruch 1 bis 4, dadurch gekennzeichnet, daß die Konzentration des eingesetzten Ammoniaks 40 bis 70 Gew.-% beträgt.

6. Verfahren nach Anspruch 1 bis 5, dadurch gekennzeichnet, daß katalytische Mengen Kupfer oder Kupferverbindungen zugesetzt werden.

7. Verfahren nach Anspruch 1 bis 6, dadurch gekennzeichnet, daß die Umsetzung kontinuierlich durchgeführt wird.

**Claims**

1. A process for preparing 2-amino-5-nitrotoluene, wherein 2-chloro-5-nitrotoluene is reacted with aqueous ammonia which has a concentration of at least 30 % by weight.

2. The process as claimed in claim 1, wherein the reaction takes place at 180 - 250° C.

3. The process as claimed in claims 1 and 2, wherein the reaction takes place at 200 - 220° C.

4. The process as claimed in claims 1 to 3, wherein the concentration of the ammonia used is up to 80 % by weight.

5. The process as claimed in claims 1 to 4, wherein the concentration of the ammonia used is 40 to 70 % by weight.

6. The process as claimed in claims 1 to 5, wherein catalytic amounts of copper or of copper compounds are added.

7. The process as claimed in claims 1 to 6, wherein the reaction is carried out continuously.

**Revendications**

1. Procédé de préparation de l'amino-2 nitro-5 toluène, procédé caractérisé en ce qu'on fait réagir le chloro-2 nitro-5 toluène avec de l'ammoniaque aqueuse d'une concentration d'au moins 30 % en poids.

2. Procédé selon la revendication 1 caractérisé en ce que la réaction est effectuée à une température de 180 à 250° C.

3. Procédé selon l'une des revendications 1 et 2 caractérisé en ce que la réaction est effectuée à une température de 200 à 220°C.

4. Procédé selon l'une quelconque des revendications 1 à 3 caractérisé en ce que la concentration de l'ammoniaque mise en jeu peut aller jusqu'à 80 % en poids.

5. Procédé selon l'une quelconque des revendications 1 à 4 caractérisé en ce que la concentration de l'ammoniaque mise en jeu est comprise entre 40 et 70 % en poids.

6. Procédé selon l'une quelconque des revendications 1 à 5 caractérisé en ce qu'on ajoute des quantités catalytiques de cuivre ou de composés du cuivre.

7. Procédé selon l'une quelconque des revendications 1 à 6 caractérisé en ce qu'on effectue la réaction en continu.